# EUROPEAN PATENT SPECIFICATION

(11) **EP 3 167 919 B1**
(45) Date of publication and mention of the grant of the patent: **31.03.2021**
(21) Application number: 14897191.4
(22) Date of filing: 08.07.2014
(51) Int. Cl.: A61M 1/16, A61M 1/18, B01D 63/02, B01D 69/02

(54) **BLOOD PURIFIER**
BLUTREINIGER
ÉPURATEUR DE SANG

(43) Date of publication of application: 17.05.2017
(73) Proprietor: University of Yamanashi, Kofu-shi, Yamanashi 400-8510 (JP); School Juridical Person The Kitasato Institute, Tokyo 108-8641 (JP); Nipro Corporation, Osaka-shi, Osaka 531-8510 (JP)
(72) Inventor: MATSUDA, Kenichi, Kofu-shi Yamanashi 400-8510 (JP); KOKUBO, Kenichi, Sagamihara-shi Kanagawa 252-0373 (JP); KOBAYASHI, Hirosuke, Sagamihara-shi Kanagawa 252-0373 (JP); SUNOHARA, Takashi, Osaka-shi Osaka 531-8510 (JP); NOTAZAWA, Shunsuke, Osaka-shi Osaka 531-8510 (JP); FUKUSHIMA, Hiroshi, Osaka-shi Osaka 531-8510 (JP)
(74) Representative: Bertsch, Florian Oliver
(86) International application number: PCT/JP2014/068213
(87) International publication number: WO 2016/006041

(56) References cited:
- WO-A1-2013/118859
- JP-A- H1 080 477
- JP-A- H1 080 477
- JP-A- H06 228 887
- JP-A- 2006 340 977
- US-A- 6 013 182

## Description

### TECHNICAL FIELD

The present invention relates to a blood purifier used for a dialysis therapy.

### BACKGROUND ART

Japanese Patent Laying-Open No. 10-080477 (Patent Document 1) discloses an invention relating to a module for a blood treatment. This module for a blood treatment includes a plurality of hollow fiber membranes. Each of the hollow fiber membranes is composed of a hydrophilized macromolecule and set to have an average inner diameter of 50 µm or more and less than 180 µm. The publication sets forth that such a module for a blood treatment can be used as a high-performance and/or small-sized module for a blood treatment for hemodialysis, hemofiltration, hemodiafiltration, and plasmapheresis and it is also useful as a module for at-home dialysis, a portable artificial kidney, and an implanted-type artificial kidney.

WO 2010/143647 (Patent Document 2) discloses an invention relating to hollow fiber membranes. Each of the hollow fiber membranes is formed of an ionic polymer selected from a cationic polymer or an anionic polymer. The ionic polymer comprises either a polyvinyl alcohol containing an ionic group selected from a cationic group and an anionic group or a mixture comprising a polymer containing the ionic group and a polyvinyl alcohol that does not contain the ionic group. The publication sets forth that such hollow fiber membranes allow a large ion permeation flux and also have a large selectivity coefficient and are also excellent in mechanical strength, and accordingly, allow efficient dialysis.
US 6,013,182 discloses a selectively permeable membrane for blood dialysis.

### CITATION LIST

### PATENT DOCUMENTS

Patent Document 1: Japanese Patent Laying-Open No. 10-080477
Patent Document 2: WO 2010/143647

### SUMMARY OF INVENTION

### TECHNICAL PROBLEM

In recent years, a development of a blood purifier has been considered by miniaturizing the blood purifier to be applicable to a patient with an advanced disease and a child with a small body weight, enhanced in portability, etc. to thus have higher convenience.

An object of the present invention is to provide a blood purifier which can be used for a long period of time even when it is miniaturized and has less fouling.

### SOLUTION TO PROBLEM

The object is achieved by the feature of claim 1.

A blood purifier based on the present invention comprises: a hollow fiber membrane bundle including a plurality of hollow fiber membranes having an effective length of 10 mm or more and 150 mm or less, allowing blood to flow inside the hollow fiber membranes and allowing a fluid including at least one of a dialysing fluid and a filtrated fluid to flow outside the hollow fiber membranes; and a body case accommodating the hollow fiber membrane bundle therein, a maximum value in the hollow fiber membrane bundle of a permeation flux Jv obtained by dividing a volume of a permeate fluid permeating through the hollow fiber membranes by a membrane area of the hollow fiber membranes and time, being represented as Jvmax, a linear velocity of blood supplied into the hollow fiber membrane and flowing in the hollow fiber membrane being represented as uB, a pressure difference between the blood flowing inside the hollow fiber membranes and the fluid flowing outside the hollow fiber membranes being represented as TMP, and when a filling blood volume into the hollow fiber membranes per unit membrane area of the hollow fiber membranes is 20 mL/m2 or more and 35 mL/m² or less and Jvmax/uB has a value of 0.00015 or more and 0.0006 or less, satisfied being a condition in which a TMP change rate has a value of 0.95 or more and 1.05 or less.

Preferably, the effective length of the plurality of hollow fiber membranes is 10 mm or more and 40 mm or less. Preferably, when the filling blood volume into the hollow fiber membranes per unit membrane area of the hollow fiber membranes is 20 mL/m² or more and 30 mL/m² or less and Jvmax/uB has a value of 0.00015 or more and 0.0006 or less, satisfied is a condition in which a TMP change rate has a value of 0.95 or more and 1.05 or less.

Preferably, a pressure loss caused between a blood inlet side of the plurality of hollow fiber membranes and a blood outlet side of the plurality of hollow fiber membranes is 0 mmHg or more and 60 mmHg or less. Preferably, a filling blood volume into the blood purifier is 5 mL or more and 100 mL or less.

### ADVANTAGEOUS EFFECTS OF INVENTION

The present invention can thus provide a blood purifier which can be used for a long period of time even when it is miniaturized.

### BRIEF DESCRIPTION OF DRAWINGS

Fig. 1 is a diagram schematically showing a blood purification system in an embodiment.
Fig. 2 is a diagram representing a variation of a filtration flow rate as time elapses, indicating an experimental condition of an exemplary experiment based on an embodiment.
Fig. 3 is a diagram representing a variation of a TMP as time elapses, indicating an experimental condition of an exemplary experiment based on an embodiment.
Fig. 4 is a cross section schematically showing a hollow fiber membrane used in a blood purification system in an embodiment.
Fig. 5 represents a relationship between the TMP [mmHg] and a filtration flow rate [mL/min], indicating an experimental result of an exemplary experiment based on an embodiment.
Fig. 6 represents a relationship between Jvavg [µm/s] and a TMP change rate, indicating an experimental result of an exemplary experiment based on an embodiment.
Fig. 7 represents a relationship between Jvavg/uB and the TMP change rate, indicating an experimental result of an exemplary experiment based on an embodiment.
Fig. 8 represents a relationship between Jvmax/a wall shear rate and the TMP change rate, indicating an experimental result of an exemplary experiment based on an embodiment.
Fig. 9 represents a relationship between Jvmax and the TMP change rate, indicating an experimental result of an exemplary experiment based on an embodiment.
Fig. 10 is a first diagram representing a relationship between Jvmax/uB and the TMP change rate, indicating an experimental result of an exemplary experiment based on an embodiment.
Fig. 11 is a second diagram representing a relationship between Jvmax/uB and the TMP change rate, indicating an experimental result of an exemplary experiment based on an embodiment.

### DESCRIPTION OF EMBODIMENTS

An embodiment based on the present invention will now be described hereinafter with reference to the drawings. Note that in the description of the embodiment, when numbers, amounts and the like are referred to, the present invention is not necessarily limited thereto unless otherwise indicated. In describing the embodiment, identical and corresponding components are identically denoted and may not be described repeatedly.

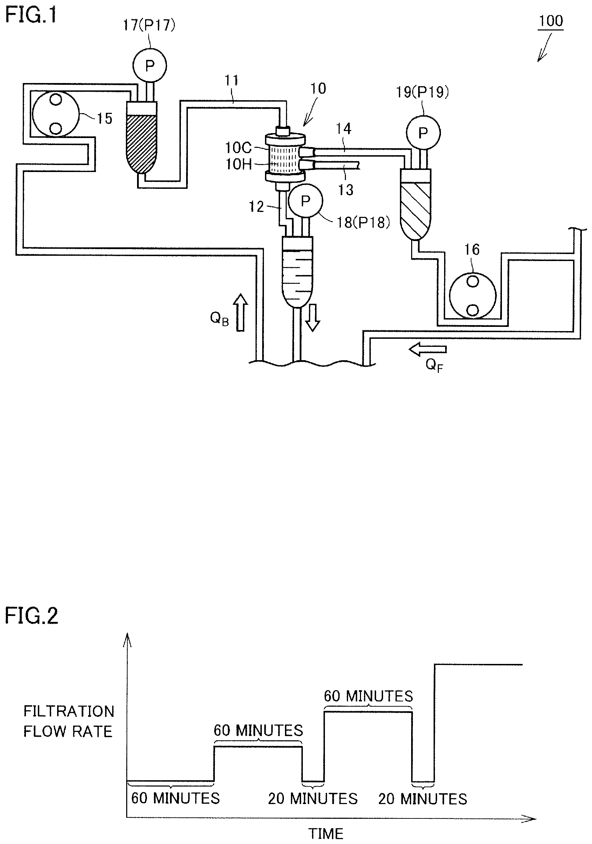

Fig. 1 is a diagram schematically showing a blood purification system 100 in an embodiment. Blood purification system 100 purifies blood inside a subject (not shown) subjected to blood purification. Blood purification system 100 can perform blood purification by an effect such as hemodialysis and hemodiafiltration and for example can be used for a treatment of a patient having an impaired renal function (a patient affected with renal insufficiency or the like and thus having an impaired function to remove wastes in blood).

Blood purification system 100 includes a blood purifier 10, blood channels 11 and 12, dialysing fluid channels 13 and 14, roller pumps 15 and 16, and monitoring pumps 17, 18, and 19. Blood purifier 10 includes a cylindrical body case 10C and a hollow fiber membrane bundle 10H accommodated in body case 10C. Hollow fiber membrane bundle 10H is composed by bundling a plurality of hollow fiber membranes. Body case 10C is provided with four ports, i.e., a blood inlet, a blood outlet, a dialysing fluid inlet, and a dialysing fluid outlet.

Connected to each port of the blood inlet and the blood outlet are blood channels 11 and 12, respectively, in communication with an inside of the plurality of hollow fiber membranes. Connected to each port of the dialysing fluid inlet and the dialysing fluid outlet are dialysing fluid channels 13 and 14, respectively, in communication with an outside of the plurality of hollow fiber membranes. Blood flows to the inside of the hollow fiber membranes through blood channel 11 connected to body case 10C. Blood flows from the inside of the hollow fiber membranes to blood channel 12. The dialysing fluid flows to the outside of the hollow fiber membranes through dialysing fluid channel 13 connected to body case 10C. The dialysing fluid flows from the outside of the hollow fiber membranes to dialysing fluid channel 14. Alternatively, channel 13 may be closed and a filtrated fluid may be discharged from channel 14.

Via the hollow fiber membranes serving as semi-permeable membranes, substances in blood (e.g., urea, creatinine, uric acid, low molecular weight protein, water, etc. accumulated in blood) move by an effect of diffusion into the dialysing fluid as a permeate fluid. The hollow fiber membranes used for blood purifier 10 are excellent in substance permeability and water permeability, and even when forced filtration by water removal is not performed, filtration (internal filtration) and back filtration (internal back filtration) can be performed between blood and the dialysing fluid via the hollow fiber membranes. Blood purifier 10 may be one which performs hemodialysis and simultaneously applies a negative pressure on the dialysing fluid's side to move substances in blood (e.g., urea, creatinine, water, etc.) to the dialysing fluid's side through filtration (i.e., hemodialysis), or may be one which furthermore performs fluid replacement (i.e., hemofiltration and hemodiafiltration).

Inside blood purifier 10, blood and the dialysing fluid are passed in mutually opposite directions. Via the hollow fiber membranes, a fluid (a permeate fluid) moves from one of blood and the dialysing fluid having higher pressure to the other having lower pressure. Internal filtration easily occurs in blood purifier 10 on the blood ingress side, and internal back filtration easily occurs in blood purifier 10 on the blood egress side.

A flow rate Q_{B} per unit time of blood flowing into blood purifier 10 can be changed by roller pump 15 provided to blood channel 11. A pressure (P17) of blood flowing into blood purifier 10 is monitored via monitoring pump 17 provided to blood channel 11. A pressure (P18) of blood flowing out of blood purifier 10 is monitored via monitoring pump 18 provided to blood channel 12.

A flow rate per unit time of the dialysing fluid flowing into blood purifier 10 can be changed by roller pump 16 provided to dialysing fluid channel 14. Through dialysing fluid channel 14, filtration of filtration flow rate Q_{F} (a water removal flow rate) is performed. A pressure (P19) on the side of the dialysing fluid is monitored by monitoring pump 19 provided to dialysing fluid channel 14. When a pressure difference between the blood flowing inside the plurality of hollow fiber membranes and the dialysing fluid flowing outside the plurality of hollow fiber membranes is represented as TMP (transmembrane pressure), the expression of TMP = (P17 + P18)/2 - P19 is established.

The plurality of hollow fiber membranes accommodated in body case 10C have an effective length of 10 mm or more and 150 mm or less, and miniaturization etc. is achievable by this dimensional range. The effective length as referred to herein is a length of a portion of the plurality of hollow fiber membranes, as seen in the longitudinal direction thereof, that is actually used to exchange substances between the blood's side and the dialysis's side.

The plurality of hollow fiber membranes have their longitudinal opposite ends fixed to an internal wall of body case 10C via a sealing resin. The effective length of the plurality of hollow fiber membranes indicates an average length of a portion of the plurality of hollow fiber membranes excluding the opposite ends fixed to body case 10C (or the sealing resin), for example. When using blood purification system 100 for portable blood purification is assumed, shortening a blood circuit is advantageous in view of the pumping performance and the filling blood volume.

Ideally blood purifier 10 is fixed at a position close to the body using a belt etc. Blood purifier 10 having a large length would limit a patient's activity and is thus not preferable, and it is desirable to wind blood purifier 10 of a maximally short length around the torso of the patient laterally (or horizontally).

An adult's torso has a width of about 200 mm and a child's torso has a width of about 150 mm. When the shape of the port of body case 10C is considered the plurality of hollow fiber membranes with an effective length of 10 mm or more and 150 mm or less allows blood purifier 10 to be fixed to the body of the patient in close contact therewith without substantially limiting the patient's activity. In view of further miniaturization etc., the effective length of the plurality of hollow fiber membranes is preferably 10 mm or more and 100 mm or less, more preferably 10 mm or more and 40 mm or less.

### [Exemplary Experiment]

When blood contacts the hollow fiber membranes, each of the hollow fiber membranes has a surface deformed, a blood component, protein etc. deposit on the surface of the hollow fiber membrane and a gel layer is thus formed thereon, adsorption causes stricture or occlusion of pores of the membrane (i.e., fouling), blood coagulates and thrombus is also formed in the hollow fiber membrane's lumen itself, etc. Various setting conditions, such as a blood flow rate, a filtration flow rate, and a ratio between the blood flow rate and the filtration flow rate, have been studied so that when hemofiltration (C:Continuous) HF is carried out over a long period of time using the above described blood purification system 100 (see Fig. 1), fouling, coagulation, etc. less easily occur on the hollow fiber membranes of blood purifier 10 and the performance of blood purifier 10 can be exhibited appropriately. In this exemplary experiment, blood purifier 10 which has a configuration of an example based on the above described embodiment, and a blood purifier which has a configuration of a comparative example different therefrom were prepared.

### (Examples)

A material of a member which configures body case 10C based on the example was PC (polycarbonate). Body case 10C had a body having an inner diameter set to 40.0 mm. Body case 10C had an overall length set to 52.6 mm. In the present example, the hollow fiber membranes had an effective length set to 37.6 mm (in average value). A material of a member which configures each of the hollow fiber membranes was CTA (cellulose triacetate).

The hollow fiber membranes had an inner diameter set to 100 µm (in average value). The hollow fiber had a membrane thickness set to 15 µm (in average value). The hollow fiber had a membrane area set to 0.55 m² (in average value). The membrane area as referred to herein is an area of a portion of the hollow fiber membrane, as seen in the longitudinal direction thereof, that is actually used to exchange substances between the blood's side and the dialysis's side, and it is a value calculated with reference to the hollow fiber's internal surface. The membrane area of the hollow fiber membrane as referred to herein is a membrane area, as averaged, of a portion of the plurality of hollow fiber membranes excluding the opposite ends fixed to body case 10C (or the sealing resin).

46,228 hollow fiber membranes were bundled together. A ratio (L/D) of the effective length (L) of the hollow fiber membranes and an average inner diameter (D) of the body of body case 10C was set to 0.94 (in average value). The hollow fiber membranes had a lumen having a cross-sectional area set to 363 mm² (in average value). A proportion of the hollow fiber membranes (the hollow fiber membrane bundle) occupying the volume of body case 10C (i.e., a fiber density ratio) was set to 49%. A general blood purifier has a so-called elongate shape. Blood purifier 10 used in the present example has a short and thick shape.

A filling blood volume into the hollow fiber membranes per unit membrane area of the hollow fiber membranes (in average value) is set to 20 mL/m² or more and 35 mL/m² or less. A filling blood volume into the hollow fiber membranes per unit membrane area of the hollow fiber membranes (in average value) is preferably 20 mL/m² or more and 30 mL/m² or less. For a given membrane area, a smaller filling blood volume allows better filtration efficiency and also allows a reduced degree of reduction in performance, and accordingly, smaller filling blood volumes are better. When converted into a given filling blood volume, it can be said that a larger corresponding membrane area allows better filtration efficiency and also allows a reduced degree of reduction in performance. In other words, a smaller filling blood volume into the hollow fiber membrane (in other words, a hollow fiber membrane having a smaller inner diameter) is advantageous in hemofiltration. In the present example, a filling blood volume into the hollow fiber membranes per unit membrane area of the hollow fiber membranes (in average value) was set to 25 mL/m².

A priming volume (a filling blood volume) into blood purifier 10 is set to 5 mL or more and 100 mL or less. A priming volume (a filling blood volume) into the hollow fiber membrane bundle in blood purifier 10 is preferably 5 mL or more and 80 mL or less, more preferably 5 mL or more and 50 mL or less. Generally, it is said that an intracorporeal circulating blood volume is 7 to 8% of a body weight. When a patient has a body weight of 20 kg, his/her intracorporeal circulating blood volume is 20000 x 0.07 = 1500 [g].

It is supposed that it is necessary to set an extracorporeal circulating blood volume in a blood purification therapy to 10% or less of an intracorporeal circulating blood volume. A blood volume allowed to extracorporeally circulate for the above case will be 1500 x 0.10 = 150 [g] at the maximum. When blood's specific gravity is taken into consideration, the blood volume allowed to extracorporeally circulate will be 150 x 0.95 = 142.5 [mL].

When blood purifier 10 is assumed to be used for portable blood purification with a blood circuit adopted which has a filling blood volume of a minimum class, an extracorporeal circulating blood volume for example of about 40 mL will be required. In that case, the filling blood volume of the blood purifier must be 142.5 - 40 = 102.5 [mL] or less. Furthermore, when blood purifier 10 is assumed to be used for portable blood purification, using a blood product etc. is hardly assumed, and accordingly, 102.5 x 0.50 = 51.25 [mL] or less would be desirable. In the present example, the priming volume (or filling blood volume) into blood purifier 10 was set to 34.7 mL.

It is recommendable that a pressure loss caused between the blood inlet side of the hollow fiber membranes and the blood outlet side of the hollow fiber membranes (in average value) be 0 mmHg or more and 60 mmHg or less. Having a structure with a smaller pressure loss allows a smaller load on blood. In other words, for a given membrane area, a short and thick module is more advantageous for hemo filtration than an elongate module. In the present example, a pressure loss caused between the blood inlet side of the hollow fiber membranes and the blood outlet side of the hollow fiber membranes (in average value) was set to 56 mmHg for an example 1, 27 mmHg for an example 2, and 19 mmHg for an example 3. As a subject to the experiment, 1L of blood of a swine was prepared. This blood had an HCT adjusted to 30% and a TP adjusted to 6.5 g/dL.

### (Comparative example)

A material of a member which configures a body case based on a comparative example was PC (polycarbonate). The body case had a body having an inner diameter set to 23.7 mm. The body case had an overall length set to 183 mm. In the comparative example, the hollow fiber membranes had an effective length set to 165 mm (in average value). A material of a member which configures each of the hollow fiber membranes was CTA (cellulose triacetate).

The hollow fiber membranes had an inner diameter set to 200 µm (in average value). The hollow fiber had a membrane thickness set to 15 µm (in average value). The hollow fiber had a membrane area set to 0.52 m² (in average value). The membrane area as referred to herein is an area of a portion of the hollow fiber membrane, as seen in the longitudinal direction thereof, that is actually used to exchange substances with the blood's side, and it is a value calculated with reference to the hollow fiber's internal surface. The membrane area of the hollow fiber membranes as referred to herein is a membrane area, as averaged, of a portion of the plurality of hollow fiber membranes excluding the opposite ends fixed to the body case (or the sealing resin).

4,992 hollow fiber membranes were bundled together. A ratio (L/D) of the effective length (L) of the hollow fiber membranes and an average inner diameter (D) of the body of the body case was set to 6.96 (in average value). The hollow fiber membranes had a lumen having a cross-sectional area set to 157 mm² (in average value). A proportion of the hollow fiber membranes (the hollow fiber membrane bundle) occupying the volume of the body case (i.e., a fiber density ratio) was set to 47%. The present comparative example's blood purifier has a so-called elongate shape.

In the present comparative example, a pressure loss caused between the blood inlet side of the hollow fiber membranes and the blood outlet side of the hollow fiber membranes (in average value) was set to 105 mmHg for a comparative example 1, 50 mmHg for a comparative example 2, and 32 mmHg for a comparative example 3. As a subject to the experiment, 1L of blood of a swine was prepared. This blood had an HCT adjusted to 30% and a TP adjusted to 6.5 g/dL.

With reference to Fig. 2, in blood purification system 100, hemofiltration was started, and from a time point at which the filtration was started, blood was circulated for 60 minutes with a fixed filtration flow rate set to perform hemofiltration. After 60 minutes from the time point at which the filtration was started, the filtration flow rate was increased by a fixed amount and in that condition, blood was circulated for 60 minutes. Subsequently, the filtration flow rate was returned to the initial filtration flow rate, and in that condition, blood was circulated for 20 minutes. Subsequently, the filtration flow rate was increased to be larger than the immediately previous value by a fixed amount, and in that condition, blood was circulated for 60 minutes. The filtration flow rate was increased by an amount set such that a ratio of the blood flow rate and the filtration flow rate was increased to be 0.025 followed by 0.05 followed by 0.075 followed by 0.1 whenever the filtration flow rate was increased.

In the above system in which, as counted from a time point at which filtration is started, after a period of 60 minutes has elapsed, a 20-minute circulation with a low filtration flow rate and a 60-minute circulation with a filtration flow rate increased by a fixed amount, as described above, are repeated, a value (in average value) of the TMP when the blood flow rate was set to 50 mL/min, a value (in average value) of the TMP when the blood flow rate was set to 100 mL/min, and a value (in average value) of the TMP when the blood flow rate was set to 200 mL/min were measured.

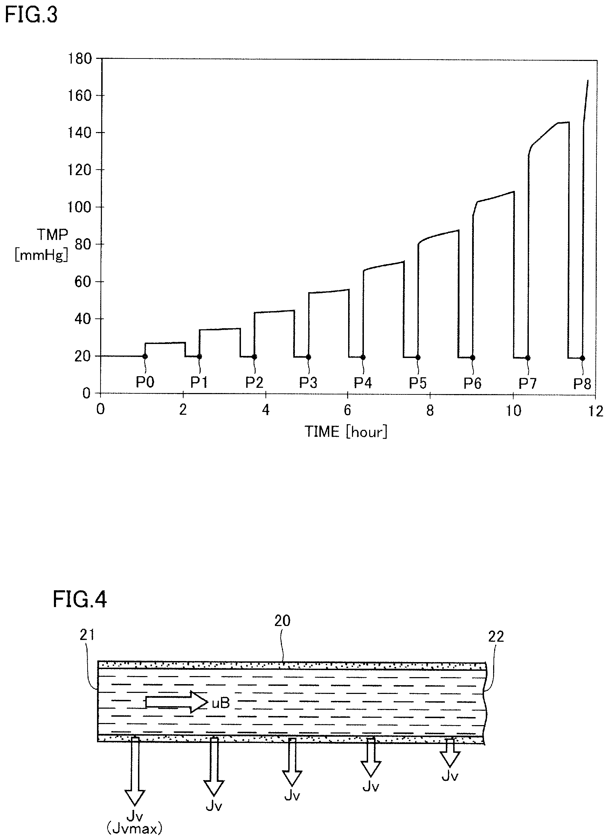

With reference to Fig. 3, the TMP was measured to obtain a value (P0) of the TMP at a time point when a period of 60 minutes has elapsed since a time point when filtration was started, and a value (P1-P8) of the TMP at a time point when the 20-minute circulation with the low filtration flow rate as described above was performed. Fig. 3 is a graph which represents a transition in value of the TMP (points P0-P8) when the blood flow rate is set to 200 mL/min. A value of the TMP at a time point when a period of 60 minutes has elapsed since filtration was started (i.e., P0) serves as a reference, and the filtration flow rate was varied and thereafter once a period of 60 minutes has elapsed the filtration flow rate was returned to the initial value, and thereafter when a period of 20 minutes has elapsed each TMP (P1-P8) was regarded as a TMP increase rate and thus calculated. The calculation was done for blood flow rates set to 50 mL/min, 100 mL/min, and 200 mL/min.

With reference to Fig. 4, a permeation flux Jv is obtained by dividing a volume of a permeate fluid permeating through hollow fiber membrane 20 by the hollow fiber membranes' membrane area and time. Permeation flux Jv can also be represented by the expression of Jv = Qf/S using a filtration flow rate Qf [µm³/s] and a membrane area S [µm²]. Permeation flux Jv presents different values at any locations in the longitudinal direction of hollow fiber membrane 20. Of permeation flux Jv, a maximum value in the hollow fiber membrane bundle is represented as Jvmax.

Generally, permeation flux Jv, as seen in the longitudinal direction of hollow fiber membrane 20, presents a larger value on the side of an inlet 21 of blood and a smaller value on the side of an outlet 22 of blood. Maximum filtration flux Jvmax will be a value equal to a value of permeation flux Jv that is obtained at a portion most upstream in the longitudinal direction of hollow fiber membrane 20, for example. The side of inlet 21 of blood is a site filtrating more blood than a downstream side (i.e., the side of outlet 22 of blood), and accordingly, a large load acts on the side of inlet 21 of blood. Noting maximum filtration flux Jvmax is effective in calculating the hollow fiber membrane's period of endurance etc.

When the hollow fiber membrane's filtration coefficient is represented as Lp [µm/(s · mmHg)], an AF differential pressure when a period of time of 60 minutes has elapsed after a flow rate was changed is represented as ΔP_{AF} [mmHg], and a colloid osmotic pressure is represented as Δπ [mmHg], then maximum filtration flux Jvmax [µm/s] can also be obtained by the expression of Jvmax = {Lp x (ΔP_{AF} - Δπ)} as a theoretical maximum permeation flux for that pressure. In this exemplary experiment, the value of filtration coefficient Lp [µm/(s · mmHg)] was corrected by the viscosity of plasma and water and set to 0.0381 for the example and 0.0637 for the comparative example, and the value of colloid osmotic pressure Δπ [mmHg] was set to 22.88.

An average value of permeation flux Jv is indicated by an average filtration flux Jvavg. When the filtration flow rate is represented as Q_{F} [µm³/s] and the hollow fiber membranes' membrane area is represented as S [µm²], average filtration flux Jvavg [µm/s] can also be obtained by the expression of Jvavg = {Q_{F}/S}. In the present exemplary experiment, filtration flow rate Q_{F} [µm³/s] had a value varied from 2.5% to 25% of the blood flow rate, and for example for a blood flow rate of 200 mL/min, it was set to 8.3 x 10¹⁰ to 8.3 x 10¹¹, and membrane area S [µm²] had a value set to 5 x 10¹¹.

### (Relationship between TMP and filtration flow rate)

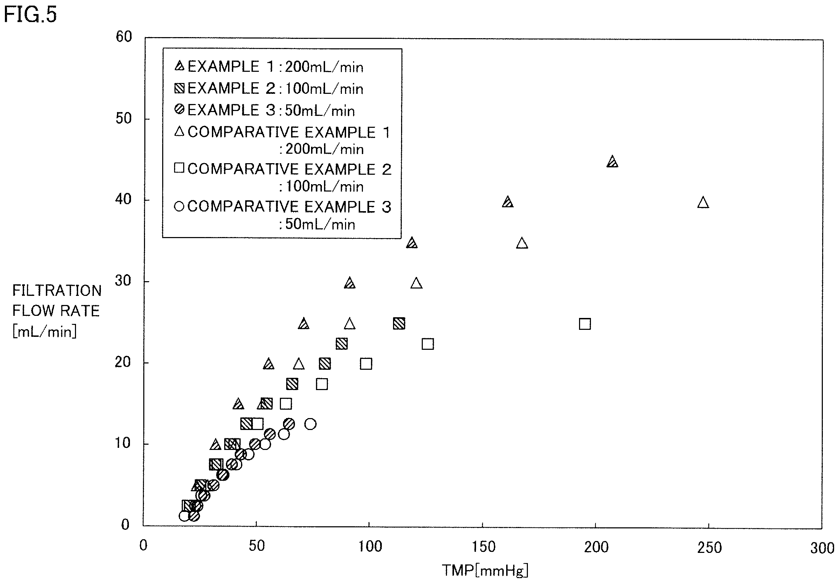

Fig. 5 represents a relationship between the TMP [mmHg] and a filtration flow rate [mL/min]. Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3.

Regarding the blood purifier having the configuration of the comparative example described above, a result when the blood flow rate of the blood purifier is set to 200 mL/min is plotted in the figure as comparative example 1. A result when the blood flow rate of the blood purifier is set to 100 mL/min is plotted in the figure as comparative example 2. A result when the blood flow rate of the blood purifier is set to 50 mL/min is plotted in the figure as comparative example 3.

For each plot, the leftmost indication in the figure corresponds to point P0 in Fig. 3. For each plot, an indication immediately adjacent thereto rightward corresponds to point P1 in Fig. 3. Similarly, indications successively adjacent rightward correspond to points P2-P8 in Fig. 3. It can be seen that the plots of Examples 1-3 and comparative examples 1-3 are such that as the filtration flow rate is increased by a fixed amount (or proceeds upward), the TMP also has an increased value.

### (Relationship between Jvavg and TMP change rate)

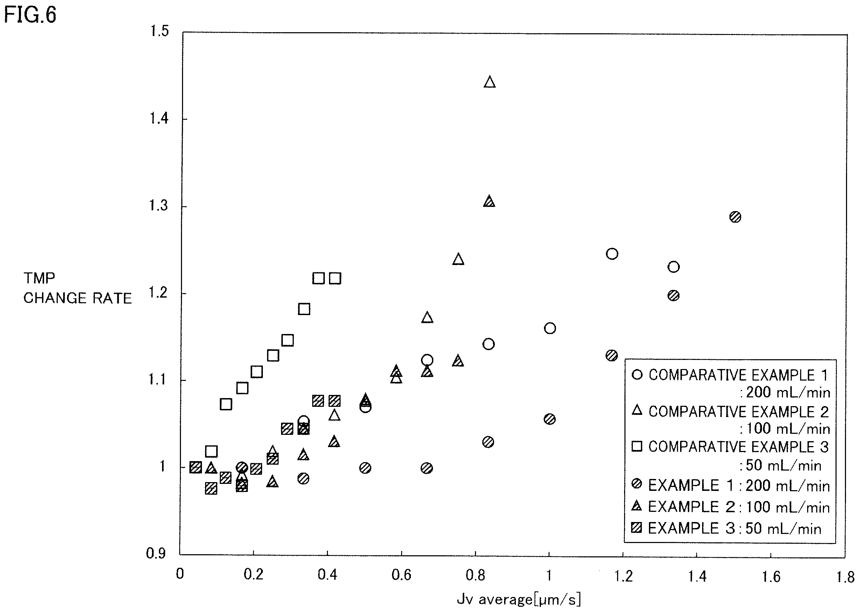

Fig. 6 shows a relationship between Jvavg [µm/s] and the TMP change rate. Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3.

Regarding the blood purifier having the configuration of the comparative example described above, a result when the blood flow rate of the blood purifier is set to 200 mL/min is plotted in the figure as comparative example 1. A result when the blood flow rate of the blood purifier is set to 100 mL/min is plotted in the figure as comparative example 2. A result when the blood flow rate of the blood purifier is set to 50 mL/min is plotted in the figure as comparative example 3.

For each plot, the leftmost indication in the figure corresponds to a TMP change rate calculated from points P0 and P1 in Fig. 3. For each plot, an indication immediately adjacent thereto rightward corresponds to a TMP change rate calculated from points P0 and P2 in Fig. 3. Similarly, indications successively adjacent rightward correspond to TMP change rates calculated from points P0 and P3-P8 in Fig. 3.

It can be seen that the plots of examples 1-3 each have a leftward indication presenting a TMP change rate assuming a value of 1.0 or in a vicinity thereof. It can be seen that as each plot proceeds rightward (or as the filtration flow rate is increased by a fixed amount), Jvavg increases and the TMP change rate also increases.

It can be seen that the plots of comparative examples 1-3 each have a leftward indication presenting a TMP change rate assuming a value of 1.0 to 1.2. It can be seen that as each plot proceeds rightward (or as the filtration flow rate is increased by a fixed amount), Jvavg increases and the TMP change rate increases more steeply than in examples 1-3.

In comparing the blood purifier of the example with the blood purifier of the comparative example, when they are compared from a viewpoint of a relationship between Jvavg and the TMP change rate, for example the transition of Example 2 and the transition of comparative example 1 present similar trends and Example 3 transitions between comparative example 2 and comparative example 3, and accordingly it can be said that they can be compared more easily from a viewpoint of a "relationship between Jvmax/uB and the TMP change rate" as described below, which will be described in detail later.

### (Relationship between Jvavg/uB and TMP change rate)

A linear velocity of blood which is supplied into the hollow fiber membrane and flows in the hollow fiber membrane is represented as uB [µm/s] (see Fig. 4). When a blood flow rate per unit time of blood flowing into the blood purifier is represented as Q_{B} [µm³/s] and a sum in cross-sectional area of the hollow fiber membranes is represented as A [µm²], then linear velocity uB can be represented by the expression of uB = Q_{B}/A.

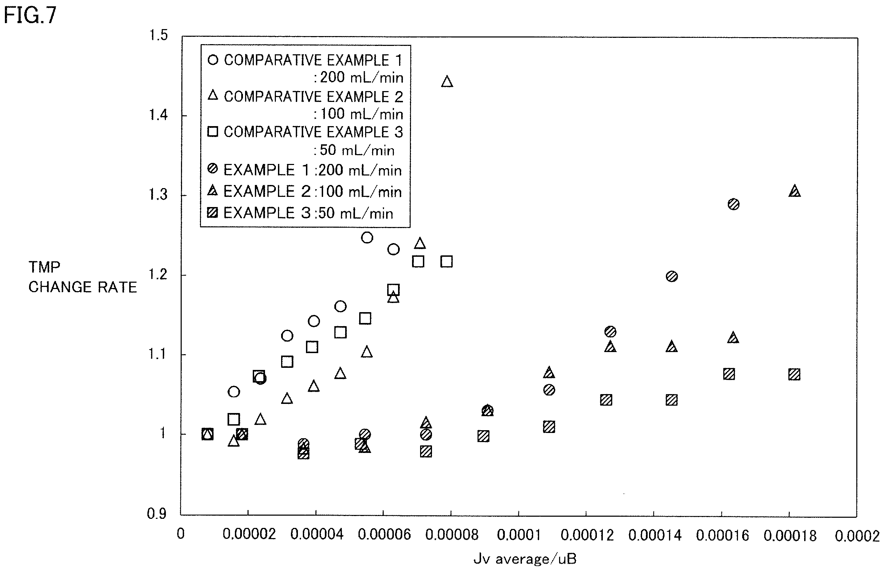

Fig. 7 shows a relationship between Jvavg/uB and the TMP change rate. Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3.

Regarding the blood purifier having the configuration of the comparative example described above, a result when the blood flow rate of the blood purifier is set to 200 mL/min is plotted in the figure as comparative example 1. A result when the blood flow rate of the blood purifier is set to 100 mL/min is plotted in the figure as comparative example 2. A result when the blood flow rate of the blood purifier is set to 50 mL/min is plotted in the figure as comparative example 3.

For each plot, the leftmost indication in the figure corresponds to a TMP change rate calculated from points P0 and P1 in Fig. 3. For each plot, an indication immediately adjacent thereto rightward corresponds to a TMP change rate calculated from points P0 and P2 in Fig. 3. Similarly, indications successively adjacent rightward correspond to TMP change rates calculated from points P0 and P3-P8 in Fig. 3.

It can be seen that the plots of examples 1-3 each have a leftward indication presenting a TMP change rate assuming a value of 1.0 or in a vicinity thereof. It can be seen that as each plot proceeds rightward (or as the filtration flow rate is increased by a fixed amount), Jvavg/uB increases and the TMP change rate also increases.

It can be seen that the plots of comparative examples 1-3 each have a leftward indication presenting a TMP change rate assuming a value of 1.0 or in a vicinity thereof. It can be seen that as each plot proceeds rightward (or as the filtration flow rate is increased by a fixed amount), Jvavg/uB increases and the TMP change rate also increases.

In comparing the blood purifier of the example with the blood purifier of the comparative example, when they are compared from a viewpoint of a relationship between Jvavg/uB and the TMP change rate, for example for Jvavg/uB in a range of 0.005-0.01 the transitions of Examples 2 and 3 and the transitions of comparative examples 1 and 2 present similar trends and for Jvavg/uB in a range of 0.01-0.015 the transition of Example 3 and the transition of comparative example 2 present similar trends etc., and accordingly, it can be said that they can be compared more easily from a viewpoint of a "relationship between Jvmax/uB and the TMP change rate" as described below, which will be described in detail later.

### (Relationship between Jvmax/a wall shear rate and TMP change rate)

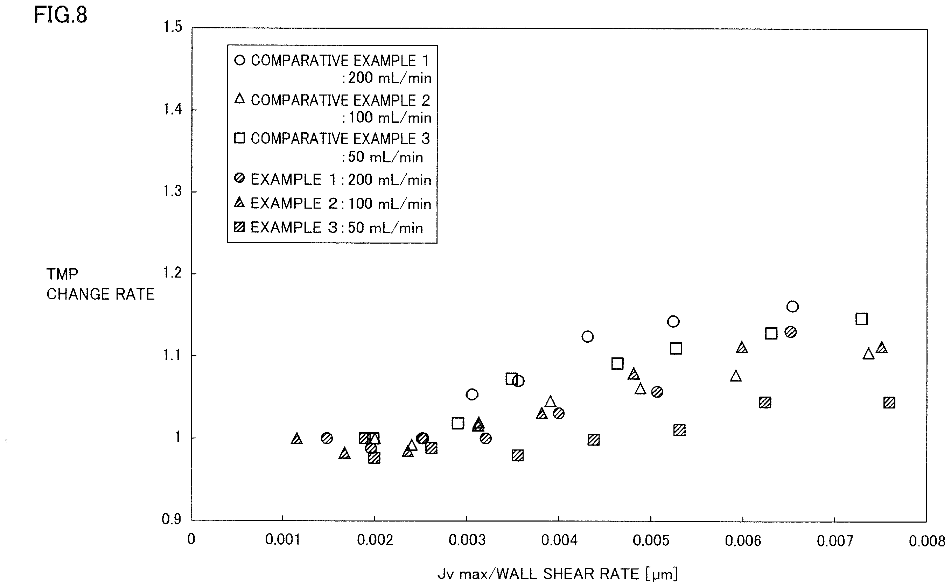

Fig. 8 shows a relationship between Jvmax/wall shear rate and the TMP change rate. The wall shear rate is a proportion at which an amount of movement of a fluid increases with time, and for example in a flow of a laminar flow of a circular tube, it can be obtained by the expression of (wall shear rate) = 4uB/R, where R represents a distance from a wall surface and uB represents the fluid's average velocity. Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3.

Regarding the blood purifier having the configuration of the comparative example described above, a result when the blood flow rate of the blood purifier is set to 200 mL/min is plotted in the figure as comparative example 1. A result when the blood flow rate of the blood purifier is set to 100 mL/min is plotted in the figure as comparative example 2. A result when the blood flow rate of the blood purifier is set to 50 mL/min is plotted in the figure as comparative example 3.

For each plot, the leftmost indication in the figure corresponds to a TMP change rate calculated from points P0 and P1 in Fig. 3. For each plot, an indication immediately adjacent thereto rightward corresponds to a TMP change rate calculated from points P1 and P2 in Fig. 3. Similarly, indications successively adjacent rightward correspond to TMP change rates per one hour calculated from points P2 and P3, P3 and P4, P4 and P5, P5 and P6, P6 and P7, and P7 and P8 in Fig. 3.

It can be seen that the plots of examples 1-3 each have a leftward indication presenting a TMP change rate assuming a value of 1.0 or in a vicinity thereof. It can be seen that as each plot proceeds rightward (or as the filtration flow rate is increased by a fixed amount), Jvmax/wall shear rate increases and the TMP change rate also increases.

It can be seen that the plots of comparative examples 1-3 each have a leftward indication presenting a TMP change rate assuming a value of 1.0 or in a vicinity thereof. It can be seen that as each plot proceeds rightward (or as the filtration flow rate is increased by a fixed amount), Jvmax/wall shear rate increases and the TMP change rate also increases.

In comparing the blood purifier of the example with the blood purifier of the comparative example, when they are compared from a viewpoint of a relationship between Jvmax/wall shear rate and the TMP change rate, the transitions of Examples 1-3 and the transitions of comparative examples 1-3 present similar trends, and accordingly, it can be said that they can be compared more easily from a viewpoint of a "relationship between Jvmax/uB and the TMP change rate" as described below, which will be described in detail later.

### (Relationship between Jvmax and TMP change rate)

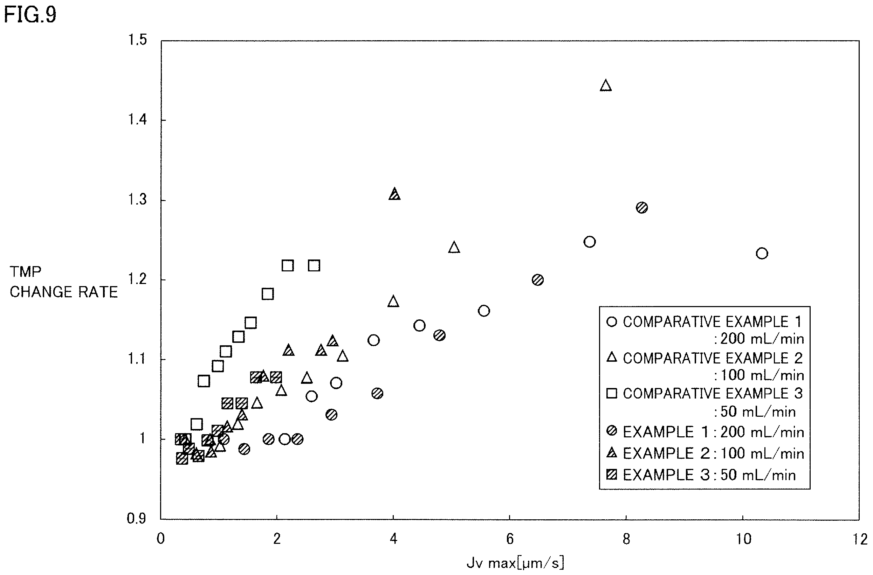

Fig. 9 shows a relationship between Jvmax and the TMP change rate. Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3. Regarding the blood purifier having the configuration of the comparative example described above, a result when the blood flow rate of the blood purifier is set to 200 mL/min is plotted in the figure as comparative example 1.

For each plot, the leftmost indication in the figure corresponds to a TMP change rate calculated from points P0 and P1 in Fig. 3. For each plot, an indication immediately adjacent thereto rightward corresponds to a TMP change rate calculated from points P1 and P2 in Fig. 3. Similarly, indications successively adjacent rightward correspond to TMP change rates per one hour calculated from points P2 and P3, P3 and P4, P4 and P5, P5 and P6, P6 and P7, and P7 and P8 in Fig. 3.

It can be seen that the plots of examples 1-3 and that of comparative example 1 each have a leftward indication presenting a TMP change rate assuming a value of 1.0 or in a vicinity thereof. It can be seen that as each plot proceeds rightward (or as the filtration flow rate is increased by a fixed amount), Jvmax increases and the TMP change rate also increases.

In comparing the blood purifier of the example with the blood purifier of the comparative example, when they are compared from a viewpoint of a relationship between Jvmax and the TMP change rate, the transitions of Examples 1-3 and the transition of comparative example 1 present similar trends. It is believed that determination of increase of the TMP is influenced by Jvmax. Regarding Example 1 (when a flow rate of blood into the blood purifier is 200 mL/min), when Jvmax exceeds 2 µm/s, an increase of the TMP is observed, and accordingly, it is believed that a value smaller than that allows use for a long period of time.

### (Relationship between Jvmax/uB and TMP change rate)

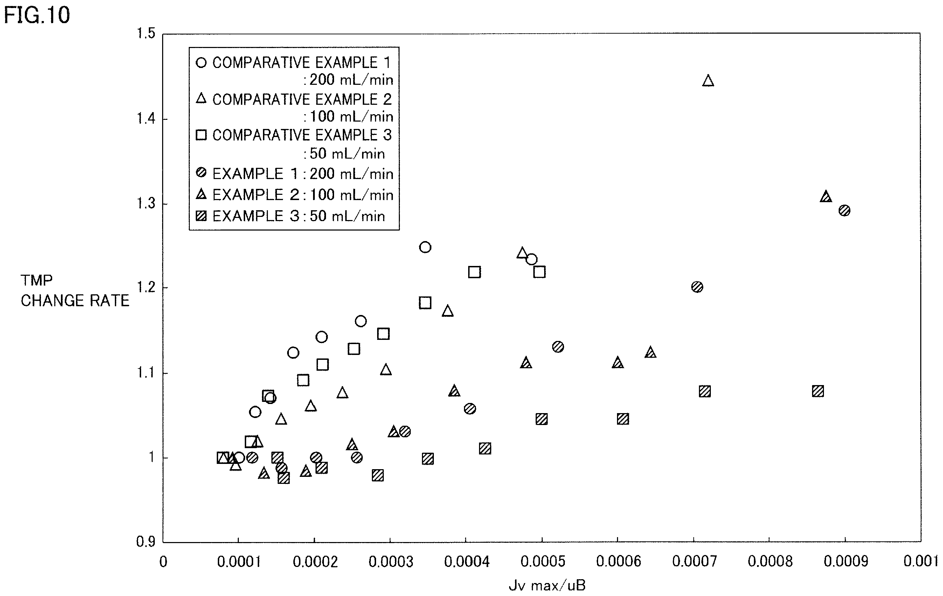

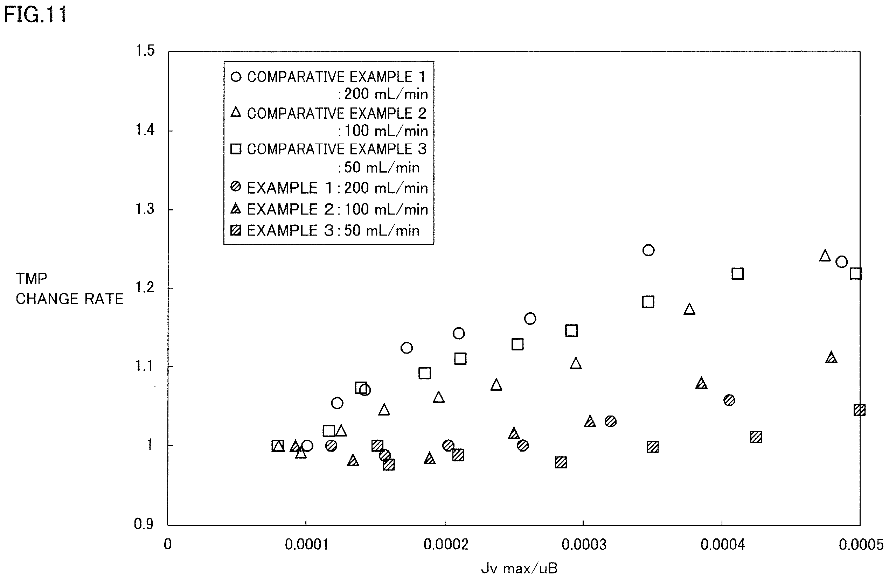

Fig. 10 and Fig. 11 are figures representing a relationship of Jvmax/uB and the TMP change rate. Fig. 11 is equivalent to an enlargement of a portion of Fig. 10 (corresponding to Jvmax/uB in a range of 0-0.0005). Regarding the blood purifier having the configuration of the example described above, a result when the blood flow rate of the blood purifier is set to 200 mL/min is plotted in the figure as Example 1. A result when the blood flow rate of the blood purifier is set to 100 mL/min is plotted in the figure as Example 2. A result when the blood flow rate of the blood purifier is set to 50 mL/min is plotted in the figure as Example 3.

Regarding the blood purifier having the configuration of the comparative example described above, a result when the blood flow rate of the blood purifier is set to 200 mL/min is plotted in the figure as comparative example 1. A result when the blood flow rate of the blood purifier is set to 100 mL/min is plotted in Fig. 11 as comparative example 2. A result when the blood flow rate of the blood purifier is set to 50 mL/min is plotted as comparative example 3.

For each plot, the leftmost indication in the figure corresponds to a TMP change rate calculated from points P0 and P1 in Fig. 3. For each plot, an indication immediately adjacent thereto rightward corresponds to a TMP change rate calculated from points P0 and P2 in Fig. 3. Similarly, indications successively adjacent rightward correspond to TMP change rates calculated from points P0 and P3-P8 in Fig. 3.

With reference to Fig. 11, it is believed that regarding Examples 1-3, if a design is done such that Jvmax is about 0.015% or more and 0.06% or less relative to linear velocity uB, the TMP change rate will assume a value of 0.95 or more and 1.05 or less even when blood purifier 10 is used for a long period of time, and it has less fouling and can be stably used. In contrast, regarding comparative examples 1-3, it can be said that even when a design is done such that Jvmax is about 0.015% or more and 0.06% or less relative to linear velocity uB, a TMP change rate when the blood purifier is used for a long period of time will assume a value exceeding 1.05, and more fouling occurs than in examples 1-3 and a range of filtration flow rate and blood flow rate allowing stable use is narrowed.

As has been set forth above, regarding examples 1-3, a filling blood volume into the hollow fiber membranes per unit membrane area of the hollow fiber membranes is set to 20 mL/m² or more and 35 mL/m² or less. Thus, it can be said that when a filling blood volume into the hollow fiber membranes per unit membrane area of the hollow fiber membranes is 20 mL/m² or more and 35 mL/m² or less and Jvmax/uB has a value of 0.00015 or more and 0.0006 or less, satisfied is a condition in which the TMP change rate has a value of 0.95 or more and 1.05 or less, and thereby, even when blood purifier 10 is used for a long period of time the TMP change rate will assume a value of 0.95 or more and 1.05 or less, and the blood purifier has less fouling and can be stably used.

More preferably it is recommendable that when a filling blood volume into the hollow fiber membranes per unit membrane area of the hollow fiber membranes is 20 mL/m² or more and 30 mL/m² or less and Jvmax/uB has a value of 0.00015 or more and 0.0006 or less, satisfied be a condition in which the TMP change rate has a value of 0.95 or more and 1.05 or less. A smaller filling blood volume into the hollow fiber membrane (in other words, a hollow fiber membrane having a smaller inner diameter) is more advantageous in hemofiltration.

From the above exemplary experiment, it can be seen that blood purifier 10 having a small filling blood volume per membrane area and having a so-called short and thick shape as described above not only allows miniaturization and enhanced portability but can also exhibit high performance when it is set under a prescribed condition and thus used.

While embodiments and examples based on the present invention have thus been described, it should be understood that the embodiments and examples disclosed herein are illustrative and non-restrictive in any respect. The scope of the present invention is defined by the terms of the claims, and is intended to include any modifications within the meaning and scope equivalent to the terms of the claims.

### REFERENCE SIGNS LIST

10: blood purifier; 10C: body case; 10H: hollow fiber membrane bundle; 11, 12: blood channel; 13, 14: dialysing fluid channel; 15, 16: roller pump; 17, 18, 19: monitoring pump; 20: hollow fiber membrane; 21: inlet; 22: outlet; 100: blood purification system.

## Claims

1. A blood purifier (10) comprising:
a hollow fiber membrane bundle (10H) including a plurality of hollow fiber membranes having an effective length of 10 mm or more and 150 mm or less, allowing blood to flow inside the hollow fiber membranes (20) and allowing a fluid including at least one of a dialysing fluid and a filtrated fluid to flow outside the hollow fiber membranes; and
a body case (10C) accommodating the hollow fiber membrane bundle therein,
a maximum value in the hollow fiber membrane bundle of a permeation flux Jv obtained by dividing a volume of a permeate fluid permeating through the hollow fiber membranes (20) by a membrane area of the hollow fiber membranes (20) and time, being represented as Jvmax,
a linear velocity of blood supplied into the hollow fiber membrane and flowing in the hollow fiber membrane being represented as uB,
a pressure difference between the blood flowing inside the hollow fiber membranes (20) and the fluid flowing outside the hollow fiber membranes (20) being represented as TMP,
when the hollow fiber membrane's filtration coefficient is represented as Lp [µm/(s · mmHg)], an AF differential pressure when a period of time of 60 minutes has elapsed after a flow rate is changed is represented as ΔP_{AF} [mmHg], and a colloid osmotic pressure is represented as Δπ [mmHg], then maximum filtration flux Jvmax [µm/s] is a value represented by the expression of Jvmax = {Lp x (ΔP_{AF} - Δπ)},
when a blood flow rate per unit time of blood flowing into the blood purifier is represented as Q_{B} [µm³/s] and a sum in cross-sectional area of the hollow fiber membranes is represented as A [µm²], then linear velocity uB is a value represented by the expression of uB = Q_{B}/A, and
when a filling blood volume into the hollow fiber membranes (20) per unit membrane area of the hollow fiber membranes is 20 mL/m² or more and 35 mL/m² or less and Jvmax/uB has a value of 0.00015 or more and 0.0006 or less, satisfied being a condition in which a TMP change rate has a value of 95% or more and 105% or less,
the TMP change rate being as determined by a case where hemofiltration is started in the blood purifier (10), and from a time point at which the filtration is started, blood is circulated for 60 minutes with a fixed filtration flow rate set to perform hemofiltration, after the 60 minutes from the time point at which the filtration is started, the filtration flow rate is increased by a fixed amount and in that condition, blood is circulated for 60 minutes, and subsequently, a 20-minute blood circulation with the filtration flow rate returned to the initial filtration flow rate and an immediately following, 60-minute blood circulation with the initial flow rate increased to be larger than the immediately previous value by a fixed amount are repeated, the fixed amount applied to increase the filtration flow rate to be larger than the immediately previous value being set such that a ratio of the blood flow rate and the filtration flow rate is increased by 0.025 whenever the filtration flow rate is increased,
a value (P0) of the TMP at a time point when a period of 60 minutes has elapsed since a time point when filtration is started, and a value (P1-P8) of the TMP at a time point when the 20-minute circulation is performed are measured, and the value (P0) of the TMP at a time point when the period of 60 minutes has elapsed since filtration was started serves as a reference, and the filtration flow rate is varied and thereafter once a period of 60 minutes has elapsed the filtration flow rate is returned to the initial value, and thereafter when a period of 20 minutes has elapsed each TMP (P1-P8) is obtained, and a ratio of each TMP (P1-P8) to the TMP (P0) serving as the reference is calculated as the TMP change rate.

2. The blood purifier (10) according to claim 1, wherein the effective length of the plurality of hollow fiber membranes (20) is 10 mm or more and 40 mm or less.

3. The blood purifier (10) according to claim 1 or 2, wherein when the filling blood volume into the hollow fiber membranes (20) per unit membrane area of the hollow fiber membranes is 20 mL/m² or more and 30 mL/m² or less and Jvmax/uB has a value of 0.00015 or more and 0.0006 or less, satisfied is a condition in which the TMP change rate has a value of 95% or more and 105% or less.

4. The blood purifier (10) according to any one of claims 1 to 3, wherein a pressure loss caused between a blood inlet side of the plurality of hollow fiber membranes and a blood outlet side of the plurality of hollow fiber membranes is 0 mmHg or more and 60 mmHg or less.

5. The blood purifier (10) according to any one of claims 1 to 4, wherein a filling blood volume into the blood purifier is 5 mL or more and 100 mL or less.

## Patentansprüche

1. Blutreiniger (10), umfassend:
ein Hohlfaser-Membranbündel (10H), das eine Vielzahl von Hohlfasermembranen mit einer effektiven Länge von 10 mm oder mehr und 150 mm oder weniger aufweist, wobei Blut innerhalb der Hohlfasermembranen (20) fließen kann und ein Fluid einschließlich mindestens eines Dialysefluids und eines filtrierten Fluids außerhalb der Hohlfasermembranen fließen können; und
ein Körpergehäuse (10C), welches das Hohlfaser-Membranbündel darin unterbringt, wobei ein maximaler Wert in dem Hohlfaser-Membranbündel eines Permeationsflusses Jv, der durch Dividieren eines Volumens eines durch die Hohlfasermembranen (20) durchdringenden Permeatfluids über eine Membranfläche der Hohlfasermembranen (20) und der Zeit, dargestellt als Jvmax, erhalten wird,
eine lineare Geschwindigkeit des in die Hohlfasermembran zugeführten und in der Hohlfasermembran fließenden Blutes, dargestellt als uB,
eine Druckdifferenz zwischen dem in den Hohlfasermembranen (20) fließenden Blut und dem außerhalb der Hohlfasermembranen (20) fließenden Fluid, dargestellt als TMP,
wenn der Filtrationskoeffizient der Hohlfasermembran als Lp [(µm / (s • mmHg)] dargestellt wird, einen AF-Differentialdruck nach Ablauf einer Zeitspanne von 60 Minuten nach Änderung einer Durchflussrate, dargestellt als ΔP_{AF} [mmHg], und einen kolloidosmotischer Druck, dargestellt als Δπ [mmHg], wobei dann der maximale Filtrationsfluss Jvmax [µm/s] ein Wert ist, der durch den Ausdruck Jvmax = {Lp x (ΔP_{AF} - Δπ)} dargestellt wird,
wenn eine Blutdurchflussrate pro Einheitszeit des in den Blutreiniger fließenden Blutes als Q_{B}. [µm³/ s] dargestellt ist und eine Summe der Querschnittsfläche der Hohlfasermembranen als A [µm²] dargestellt ist, wobei ist die Lineargeschwindigkeit uB ein Wert ist, der durch den Ausdruck uB = Q_{B}/A dargestellt wird und
wenn ein Füllblutvolumen in die Hohlfasermembranen (20) pro Einheitsmembranenfläche der Hohlfasermembranen 20 ml/m² oder mehr und 35 ml/m² oder weniger beträgt und Jvmax/uB einen Wert von 0,00015 oder mehr und 0,0006 oder weniger aufweist, eine Bedingung erfüllt ist, bei der eine TMP-Änderungsrate einen Wert von 95% oder mehr und 105% oder weniger aufweist,
die TMP-Änderungsrate, wie durch einen Fall bestimmt, in dem die Hämofiltration im Blutreiniger (10) gestartet wird, und ab einem Zeitpunkt, zu dem die Filtration gestartet wird, das Blut 60 Minuten lang mit einer festen Filtrationsdurchflussrate zirkuliert, die zur Durchführung der Hämofiltration eingestellt ist, nach den 60 Minuten, ab dem Zeitpunkt, zu dem die Filtration gestartet wird, die Filtrationsdurchflussrate um einen festen Betrag erhöht wird, und in diesem Zustand Blut 60 Minuten lang zirkuliert wird, und anschließend eine 20-minütige Blutzirkulation mit der Filtrationsdurchflussrate, die zu der anfänglichen Filtrationsdurchflussrate zurückkehrt und eine unmittelbar folgende 60-minütige Blutzirkulation mit der anfänglichen Durchflussrate erhöht wird, um um einen festen Betrag größer als der unmittelbar vorherige Wert zu sein, wiederholt werden, wobei der feste Betrag angewendet wird, um die Filtrationsdurchflussrate zu erhöhen, um größer zu sein als der unmittelbar vorherige Wert, der so eingestellt wird, dass ein Verhältnis der Blutdurchflussrate und der Filtrationsdurchflussrate um 0,025 immer dann erhöht werden, wenn die Filtrationsdurchflussrate erhöht wird,
wobei ein Wert (P0) von TMP zu einem Zeitpunkt, zu dem ein Zeitraum von 60 Minuten seit einem Zeitpunkt, als die Filtration startete, abgelaufen ist, und ein Wert (P1-P8) von TMP zu einem Zeitpunkt, zu dem die 20-Minuten-Zirkulation durchgeführt wird, gemessen wird, und der Wert (P0) von TMP zum Zeitpunkt, zu dem der Zeitraum von 60 seit der Filtration abgelaufen ist, gestartet wurde, als eine Referenz dient, und die Filtrationsdurchflussrate variiert wird und danach, sobald ein Zeitraum von 60 abgelaufen ist, die Filtrationsdurchflussrate zu dem anfänglichen Wert zurückkehrt und danach, wenn ein Zeitraum von 20 Minuten abgelaufen ist, jede TMP (P1-P8) erhalten wird und ein Verhältnis von TMP (P1-P8) zu TMP (P0), das als die Referenz dient, als die TMP-Änderungsrate berechnet wird.

2. Blutreiniger (10) nach Anspruch 1, wobei die effektive Länge der Vielzahl von Hohlfasermembranen (20) 10 mm oder mehr und 40 mm oder weniger beträgt.

3. Blutreiniger (10) nach Anspruch 1 oder 2, wobei, wenn ein Füllblutvolumen in die Hohlfasermembranen (20) pro Einheitsmembranenfläche der Hohlfasermembranen 20 ml/m² oder mehr und 30 ml/m² oder weniger beträgt und Jvmax/uB einen Wert von 0,00015 oder mehr und 0,0006 oder weniger aufweist, eine Bedingung erfüllt ist, bei der eine TMP-Änderungsrate einen Wert von 95% oder mehr und 105% oder weniger aufweist.

4. Blutreiniger (10) nach einem der Ansprüche 1 bis 3, wobei ein Druckverlust, der zwischen einer Bluteinlassseite der Vielzahl von Hohlfasermembranen und einer Blutauslassseite der Vielzahl von Hohlfasermembranen verursacht wird, 0 mmHg oder mehr und 60 mmHg oder weniger beträgt.

5. Blutreiniger (10) nach einem der Ansprüche 1 bis 4, wobei ein Füllblutvolumen in den Blutreiniger 5 ml oder mehr und 100 ml oder weniger beträgt.

## Revendications

1. Épurateur de sang (10) comprenant :
un faisceau de membranes à fibres creuses (10H) incluant une pluralité de membranes à fibres creuses ayant une longueur efficace de 10 mm ou plus et de 150 mm ou moins, permettant l'écoulement de sang à l'intérieur des membranes à fibres creuses (20) et permettant l'écoulement d'un fluide incluant au moins l'un d'un fluide de dialyse et d'un fluide filtré à l'extérieur des membranes à fibres creuses ; et
un corps de boîtier (10C) hébergeant le faisceau de membranes à fibres creuses à l'intérieur de celui-ci,
une valeur maximale dans le faisceau de membranes à fibres creuses d'un flux de perméation Jv obtenu par division d'un volume d'un fluide de perméat qui passe à travers les membranes à fibres creuses (20) par une aire de membrane des membranes à fibres creuses (20) et un temps, étant représentée par Jvmax,
une vitesse linéaire du sang alimenté dans la membrane à fibres creuses et s'écoulant dans la membrane à fibres creuses étant représentée par uB,
une différence de pression entre le sang s'écoulant à l'intérieur des membranes à fibres creuses (20) et le fluide s'écoulant à l'extérieur des membranes à fibres creuses (20) étant représentée comme TMP,
lorsque le coefficient de filtration d'une membrane à fibres creuses est représenté par Lp [µm/(s · mmHg)], une pression différentielle AF lorsqu'une période de temps de 60 minutes s'est écoulée après un changement de débit est représentée par ΔP_{AF} [mmHg], et une pression osmotique colloïdale est représentée par Δπ[mmHg], alors le flux de filtration maximal Jvmax [µm/s] est une valeur représentée par l'expression de Jvmax = {Lp x (ΔP_{AF} - Δπ[)},
lorsqu'un débit de sang par temps unitaire du sang s'écoulant dans l'épurateur de sang est représenté par Q_{B}[µm³/s] et une somme dans la zone transversale des membranes à fibres creuses est représentée par A[µm²], alors la vitesse linéaire uB est une valeur représentée par l'expression de uB = Q_{B}/A, et
lorsqu'un volume de sang de remplissage dans les membranes à fibres creuses (20) par aire de membrane unitaire des membranes à fibres creuses est 20 ml/m² ou plus et 35 ml/m² ou moins et Jvmax/uB a une valeur de 0,00015 ou plus et 0,0006 ou moins, une condition étant satisfaite dans laquelle un taux de changement TMP a une valeur de 95 % ou plus et 105 % ou moins,
le taux de changement TMP étant tel que déterminé par un cas où l'hémofiltration commence dans l'épurateur de sang (10), et à partir d'un point temporel auquel la filtration commence, le sang circule pendant 60 minutes avec un débit de filtration fixe défini pour réaliser une hémofiltration, après les 60 minutes à partir du point temporel auquel la filtration débute, le débit de filtration est accru d'une quantité fixe et dans cette condition, le sang circule pendant 60 minutes, et ensuite, une circulation de sang de 20 minutes avec le débit de filtration revenu au débit de filtration initial et une circulation de sang de 60 minutes immédiatement subséquente avec le débit initial accru pour être supérieur à la valeur immédiatement précédente d'une quantité fixe sont répétés, la quantité fixe appliquée pour faire augmenter le débit de filtration pour qu'il soit supérieur à la valeur immédiatement précédente étant telle qu'un rapport du débit de sang et du débit de filtration est accru de 0,025 chaque fois que le débit de filtration est accru,
une valeur (P0) du TMP à un point temporel auquel une période de 60 minutes s'est écoulée depuis un point temporel auquel la filtration commence, et une valeur (P1-P8) du TMP à un point temporel auquel la circulation de 20 minutes est réalisée sont mesurées, et la valeur (P0) du TMP à un point temporel auquel la période de 60 minutes s'est écoulée depuis le début de la filtration sert de référence, et le débit de filtration varie et après ceci une fois qu'une période de 60 minutes s'est écoulée le débit de filtration retourne à la valeur initiale, et après ceci lorsque une période de 20 minutes s'est écoulée chaque TMP (P1-P8) est obtenu, et un rapport de chaque TMP (P1-P8) sur le TMP (P0) servant de référence est calculé comme le taux de changement TMP.

2. Épurateur de sang (10) selon la revendication 1, dans lequel la longueur efficace de la pluralité de membranes à fibres creuses (20) est de 10 mm ou plus et de 40 mm ou moins.

3. Épurateur de sang (10) selon la revendication 1 ou 2, dans lequel lorsque le volume de sang de remplissage dans les membranes à fibres creuses (20) par aire de membrane unitaire des membranes à fibres creuses est de 20 ml/m² ou plus et de 30 ml/m² ou moins et Jvmax/uB a une valeur de 0,00015 ou plus et de 0,0006 ou moins, une condition est satisfaite dans laquelle le taux de changement TMP a une valeur de 95 % ou plus et de 105 % ou moins.

4. Épurateur de sang (10) selon l'une quelconque des revendications 1 à 3, dans lequel une perte de pression provoquée entre un côté orifice d'entrée de sang de la pluralité de membranes à fibres creuses et un côté orifice de sortie de sang de la pluralité de membranes à fibres creuses est de 0 mmHg ou plus et de 60 mmHg ou moins.

5. Épurateur de sang (10) selon l'une quelconque des revendications 1 à 4, dans lequel un volume de sang de remplissage dans l'épurateur de sang et de 5 ml ou plus et de 100 ml ou moins.
